# EUROPEAN PATENT SPECIFICATION

(11) **EP 1 977 683 B1**
(45) Date of publication and mention of the grant of the patent: **16.03.2011**
(21) Application number: 08011262.6
(22) Date of filing: 29.08.2006
(51) Int. Cl.: A61B 1/05, A61B 5/07

(54) **Intra-subject information acquiring system**
Intrakorporales Informationserfassungssystem
Système de recueil d'informations intra-sujet

(30) Priority: 29.08.2005 JP 2005248012
(43) Date of publication of application: 08.10.2008
(62) Divisional of application: 06796983.2
(73) Proprietor: Olympus Medical Systems Corp., Tokyo 151-0072 (JP); Olympus Corporation, Tokyo 151-0072 (JP)
(72) Inventor: Shigemori, Toshiaki, Tokyo 151-0072 (JP); Fujita, Manabu, Tokyo 151-0072 (JP); Nagase, Ayako, Tokyo 151-0072 (JP); Matsui, Akira, Tokyo 151-0072 (JP); Nakatsuchi, Kazutaka, Tokyo 151-0072 (JP)
(74) Representative: von Hellfeld, Axel

(56) References cited:
- US-A- 4 556 061
- US-A1- 2004 199 146
- US-A1- 2004 215 084
- US-A1- 2005 102 005
- US-A1- 2005 288 559

## Description

### TECHNICAL FIELD

The present invention relates to an intra-subject information acquiring system that notifies a remaining power amount of a body-insertable apparatus, such as a capsule endoscope, which is inserted into a subject and acquires intra-subject information.

### BACKGROUND ART

In recent years, in the field of endoscope, a capsule endoscope having an imaging function and a radio communication function has appeared. After a subject swallows the capsule endoscope from the mouth to perform an observation (examination), the capsule endoscope moves within internal organs (within the body cavity) such as an esophagus, a stomach, and a small intestine, following its peristalsis, during the observation period, until the capsule endoscope is naturally discharged from the body (human body) of the subject. The capsule endoscope sequentially images at a predetermined imaging rate, using the imaging function.

During the observation period when the capsule endoscope moves within the internal organs, image data acquired by imaging within the body cavity by the capsule endoscope is sequentially transmitted to the outside of the subject, by the radio communication function such as radio transmission, and is stored into a memory provided within an external receiving apparatus. When the subject carries the receiving apparatus having the radio communication function and the memory function, the subject can move freely even during the observation period after the subject swallows the capsule endoscope until the capsule endoscope is discharged. After the observation, a doctor or a nurse can perform diagnosis by displaying the images of the body cavity on a displaying unit such as a display, based on image data stored in the memory of the receiving device (for example, see Patent Document 1).

With regard to the supply of power, in view of the fact that the capsule endoscope works inside a living body, some battery supply systems employ a battery which is mounted in the capsule endoscope and supplies the power to the capsule endoscope, while other power transmission systems transmit power from outside the living body to the capsule endoscope.

The latter power transmission system includes a mechanism which is provided with a power receiving antenna inside and transmits the power to the capsule endoscope that remains within the living body through the power receiving antenna (see Patent Document 1).
Patent Document 1: Japanese Patent Application Laid-open No. 2001-231186

Document US 4,556,061 concerns a cardiac pacer comprising a battery and a battery consumption monitor circuit. An indication of consumed battery capacity determined by the batter consumption monitor circuit is provided via an antenna to an external readout.

Document US 2004/0215084 A1 concerns a wireless in-vivo information acquiring system and an external device. The system comprises a system control circuit controlling the drive of units provided in the system by using power stored in a capacitor and a power supply state for causing the units to drive. A level determining signal indicating a level of power received from an external power supply signal and in-vivo information are intermittently transmitted to an external device.

### DISCLOSURE OF INVENTION

### PROBLEM TO BE SOLVED BY THE INVENTION

Since some of the above power transmission systems are configured merely to supply power from the outside of the living body, however, an amount of power remaining in a capsule endoscope cannot be known.

The present invention has been achieved in view of the above, and it is an object of the invention to provide an intra-subject information acquiring system that allows a user to promptly recognize a remaining power amount of a body-insertable apparatus.

### MEANS FOR SOLVING PROBLEM

Some or all of the above problems are overcome by a body-insertable apparatus having the features of claim 1 and a receiving apparatus having the features of claim 12.

A body-insertable apparatus according to one aspect of the present invention includes: a function executing unit that executes a predetermined function of acquiring intra-subject information; a power supply unit that supplies a power to the function executing unit; a recognizing unit that recognizes a remaining power amount of the power supply unit; a superimposing unit that superimposes information of the remaining power amount recognized by the recognizing unit on a transmission signal; and a transmitting unit that transmits the transmission signal on which the information of the remaining power amount is superimposed by the superimposing unit.

A receiving apparatus according to another aspect of the present invention includes: a receiving unit that receives a transmission signal transmitted from a body-insertable apparatus, the body-insertable apparatus being inserted into a subject body and acquiring intra-subject information; a detector that detects information of a remaining power amount of the body-insertable apparatus from the transmission signal received by the receiving unit; and a display unit that displays the information of the remaining power amount detected by the detector.

### EFFECT OF THE INVENTION

According to the intra-subject information acquiring system of the present invention, a recognizing unit of a body-insertable apparatus recognizes a remaining power amount. The remaining-power-amount information is transmitted to an external device, and is displayed on a displaying unit. Therefore, there is an effect that the remaining power amount of the body-insertable apparatus can be promptly recognized.

### BRIEF DESCRIPTION OF DRAWINGS

FIG. 1 is a schematic diagram of an overall configuration of a radio intra-subject information acquiring system according to the present invention;
FIG. 2 is a block diagram of an internal configuration of a capsule endoscope according to a first embodiment of the present invention;
FIG. 3 is a block diagram of a configuration of a remaining-power-amount recognizing circuit shown in FIG. 2 and its periphery according to the first embodiment;
FIG. 4 is a block diagram of an internal configuration of a receiving apparatus according to the first embodiment;
FIG. 5 is a block diagram of a configuration of relevant parts of an external device including an RF receiving unit; and
FIG. 6 is a block diagram of a configuration of a remaining-power-amount recognizing circuit and its periphery according to a second embodiment.

### EXPLANATIONS OF LETTERS OR NUMERALS

- 1: Subject
- 2: Capsule endoscope
- 3: Receiving apparatus
- 4: Display device
- 5: Portable recording medium
- 11: LED
- 12: LED driving circuit
- 13: CCD
- 14: CCD driving circuit
- 15: RF transmitting unit
- 16: RF antenna
- 17: Power receiving antenna
- 18: Separating circuit
- 19: Power reproducing circuit
- 20: Remaining-power-amount recognizing circuit
- 20a: A/D converter
- 20b: Digital processing circuit
- 20c: Inverter
- 21: Booster circuit
- 22: Capacitor
- 23: System control circuit
- 24: Control-information detecting circuit
- 31: Transmitting/receiving jacket
- 32: External device
- 39: Power supply unit
- 40: Power-supply control circuit
- 41: Oscillator
- 42: Control-information input unit
- 43: Superimposing circuit
- 44: Amplifier circuit
- 45: Switching circuit
- 46: RF receiving unit
- 46a: RF-BB converting circuit
- 46b: Binarizing circuit
- 46c: Synchronization detecting circuit
- 46d: Serial/parallel converting circuit
- 47: Image processing unit
- 48: Remaining-power-amount detecting circuit
- 49: Storage unit
- 50: Control circuit
- 51: Display unit
- A1 to An: Receiving antenna
- B1 to Bm: Power supply Antenna
- R1, R2: Resistor

### BEST MODE(S) FOR CARRYING OUT THE INVENTION

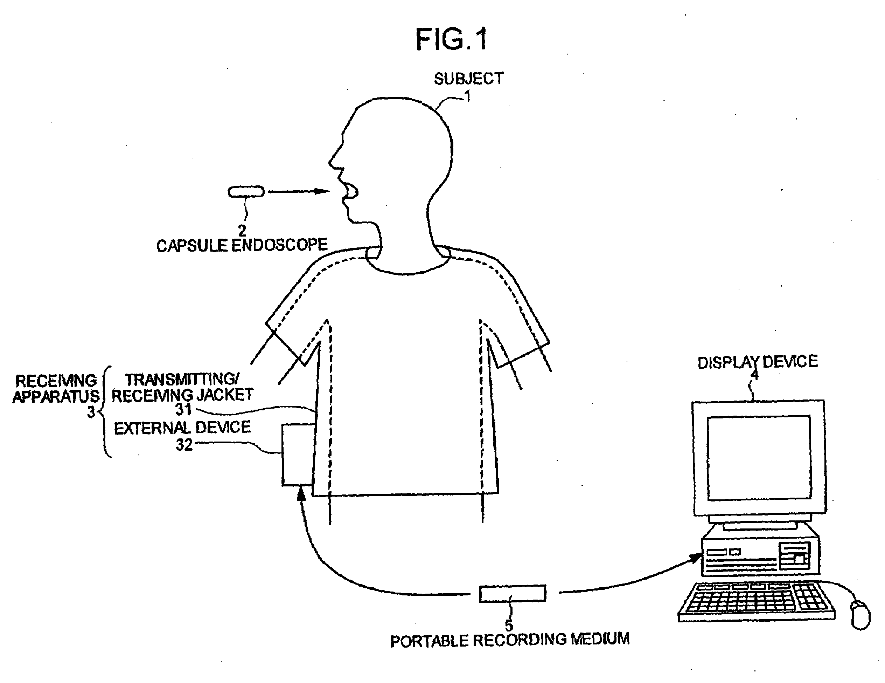

Exemplary embodiments of an intra-subject information acquiring system according to the present invention will be explained below in detail with reference to the drawings of FIG. 1 to FIG. 6. Note that the invention is not limited to the embodiments, and the embodiments can be variously modified without departing from the scope of the invention.

FIG. 1 is a schematic diagram of an overall configuration of a radio intra-subject information acquiring system according to the present invention. In the following description of the radio intra-subject information acquiring system, a capsule endoscope is explained as one example of the body-insertable apparatus. In FIG. 1, the radio intra-subject information acquiring system includes a receiving apparatus 3 that has a radio receiving function, and a capsule endoscope (a body-insertable apparatus) 2 that is inserted into a subject 1, images a body cavity, and transmits data such as an image data to the receiving apparatus 3 outside the subject 1. The radio intra-subject information acquiring system also includes a display device 4 that displays a body-cavity image based on an image signal received by the receiving apparatus 3, and a portable recording medium 5 that delivers data between the receiving apparatus 3 and the display device 4. The receiving apparatus 3 includes a transmitting/receiving jacket 31 which the subject 1 wears, and an external device 32 that processes a received radio signal.

The display device 4 displays a body-cavity image picked up by the capsule endoscope 2, and has a configuration like a workstation that displays an image based on data acquired by the portable recording medium 5. Specifically, the display device 4 can be configured to directly display an image using a CRT display, a liquid crystal display, or the like, or can be configured to output an image to other medium such as a printer.

The portable recording medium 5 is attachable to and detachable from the external device 32 and the display device 4, and can output or record information when the portable recording medium 5 is mounted on one of the external device 32 and the display device 4. In the present embodiment, while the capsule endoscope 2 moves within the body cavity of the subject 1, the portable recording medium 5 is mounted on the external device 32, and records data transmitted from the capsule endoscope 2. After the capsule endoscope 2 is discharged from the subject 1, that is, after the imaging inside the subject 1 ends, the portable recording medium 5 is taken out from the external device 32, and is mounted on the display device 4. The display device 4 reads the data recorded on the portable recording medium 5. When the portable recording medium 5 configured by a Compact Flash (Registered Trademark) memory or the like delivers data between the external device 32 and the display device 4, the subject 1 can move more freely during the imaging inside the body cavity than when the external device 32 is directly connected to the display device 4 by wire. While the portable recording medium 5 is used to deliver data between the external device 32 and the display device 4, the use of the portable recording medium 5 is not necessarily the only method. Other recording apparatuses, such as a hard disk, built in the external device 32 can be also used, and the recording apparatus and the display device 4 can be connected to each other by radio or by wire to deliver data therebetween.

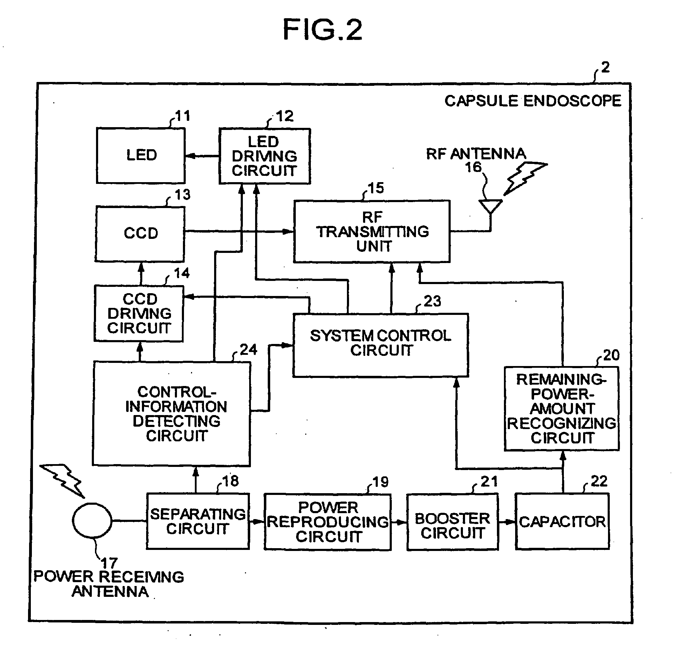

The capsule endoscope 2 is explained next. FIG. 2 is a block diagram of an internal configuration of a capsule endoscope according to the present embodiment. In FIG. 2, the capsule endoscope 2 includes: a light emitting element (LED) 11 as an illuminating unit that irradiates an examined part within a body cavity of the subject 1; an LED driving circuit 12 that controls a driving state of the LED 11; a charge-coupled device (CCD) 13 as an imaging unit that picks up an image within the body cavity as a reflection light from an area irradiated by the LED 11; a CCD driving circuit 14 that controls a driving state of the CCD 13; an RF transmitting unit 15 that modulates an image signal output from the CCD 13, into an RF signal; and an RF antenna 16 as a transmitting antenna that radio transmits an RF signal output from the RF transmitting unit 15.

The capsule endoscope 2 further includes: a power receiving antenna 17 that receives a radio signal transmitted from the receiving apparatus 3; a separating circuit 18 that separates a power supply signal from a signal received by the power receiving antenna 17; a power reproducing circuit 19 that reproduces power supply (power) from the power supply signal; a booster circuit 21 that boosts the reproduced power; a capacitor 22 that stores the boosted power supply; a remaining-power-amount recognizing circuit 20 that recognizes a remaining power amount of the capacitor 22; and a system control circuit 23 that controls each unit within the capsule endoscope 2 such as the LED 11 and the CCD 13, using power stored in the capacitor 22. In order to acquire intra-subject information, the LED 11, the CCD 13, the RF transmitting unit 15, and the system control circuit 23 execute predetermined functions of the capsule endoscope 2.

The CCD 13 acquires an intra-subject image as a result of light irradiation by the LED 11 as a light emitting element, and outputs the intra-subject image as an image signal of acquired intra-subject information, to the RF transmitting unit 15. The RF transmitting unit 15 modulates the image signal transmitted from the CCD 13, and radio transmits the modulated image signal as a transmission signal, to the outside of the capsule endoscope 2 via the RF antenna 16 as a transmission antenna. In the present invention, instead of the CCD 13, other imaging elements such as a CMOS sensor can be used. The transmission signal can be living-body information such as temperature information and pH information within the body cavity, instead of the body-cavity image of the subject.

The power receiving antenna 17 is made of a single coil member, and receives a power supply wave transmitted from the receiving apparatus 3 described latter. After the power receiving antenna 17 receives the power supply wave, the separating circuit 18 separates a power supply signal from the power supply wave. The power reproducing circuit 19 reproduces the power supply signal as a power source. The booster circuit 21 boosts the power, and stores the boosted power into the capacitor 22 as power. The system control unit 23 controls the driving of each electric device such as the LED driving circuit 12, the CCD driving circuit 14, and the RF transmission unit 15, using the power stored in the capacitor 22. The system control unit 23 also controls a power supply state to drive each electric device.

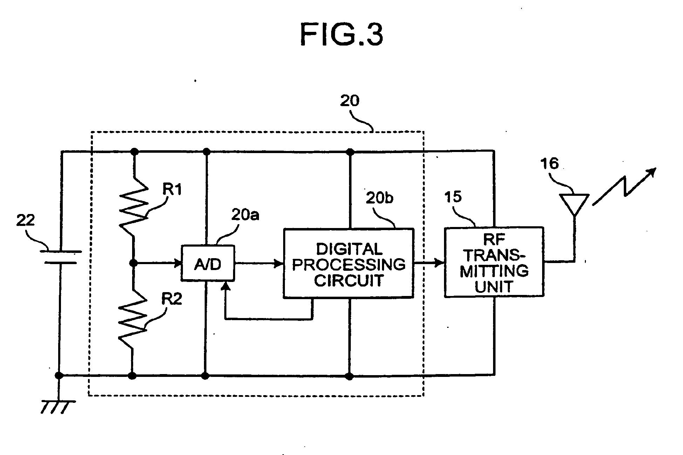

Next, the remaining-power-amount recognizing circuit 20 is explained. FIG. 3 is a block diagram showing a configuration not according to the invention of the remaining-power-amount recognizing circuit 20 shown in FIG. 2 and its periphery. In FIG. 3, the remaining-power-amount recognizing circuit 20 includes resistors R1 and R2 that resistance-divides the voltage of the capacitor 22, and an A/D converter 20a that converts the resistance-divided voltage from an analog signal into a digital signal, and a digital processing circuit 20b that digitally processes a voltage signal from the A/D converter 20a, superimposes the digital signal on the transmission signal, and outputs the superimposed signal to the RF transmitting unit 15.

The digital processing circuit 20b can superimpose only the voltage signal from the A/D converter 20a on a transmission signal, or can superimpose the voltage signal together with white-balance information and a capsule ID (identification signal) on a transmission signal. The transmission signal on which the voltage signal is superimposed can be an image signal, or can be any living-body information radio transmitted from the capsule endoscope 2 to the receiving apparatus 3.

The digital processing circuit 20b can also timer-control the starting of the A/D converter 20a so as to intermittently start the A/D converter 20a to take in a voltage signal, in addition to achieving the function of superimposing the voltage signal on the transmission signal. Based on this configuration, recognition precision of remaining power amount increases, and power consumption of the capacitor 22 can be decreased based on the intermittent control.

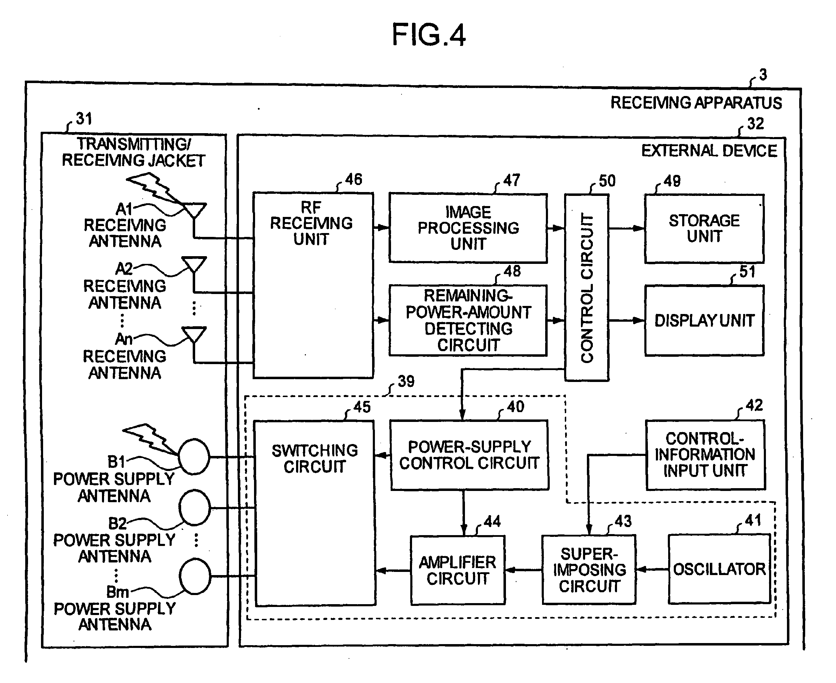

The receiving apparatus 3 is explained next. FIG. 4 is a block diagram of an internal configuration of the receiving apparatus 3 according to the embodiment. In FIG. 4, the receiving apparatus 3 has a shape to enable the subject 1 to wear the receiving apparatus 3, and includes the transmitting/receiving jacket 31 having receiving antennas A1 to An, and the external device 32 that processes a radio signal received via the transmitting/receiving jacket 31. The receiving antennas A1 to An are detachably connected to a connector not shown. The receiving antennas A1 to An can be directly adhered to the external surface of the subject 1, and are not necessarily be provided on the transmitting/receiving jacket 31, and are detachable from the transmitting/receiving jacket 31.

The transmitting/receiving jacket 31 includes the receiving antennas A1 to An, and plural power supply antennas B1 to Bm. The symbols n and m denote natural numbers. Each antenna of the transmitting/receiving jacket 31 is disposed at a predetermined position on the external surface of the subject 1 when the antenna is mounted on the living body as the subject 1. When intra-subject information is to be acquired from one or all of an esophagus, a stomach, a small intestine, and a large intestine, the transmitting/receiving jacket 31 is disposed on the breast or abdomen, or across both the breast and abdomen.

The external device 32 includes an RF receiving unit 46 that executes a predetermined signal processing such as a demodulation of radio signals received by the receiving antennas A1 to An, and that extracts image information acquired by the capsule endoscope 2 from the radio signals, an image processing unit 47 that executes necessary image processing to the extracted image information, a storage unit 49 as a recording unit that records the image-processed image information, and a control circuit 50 that controls each unit. The external device 32 performs signal processing of a radio signal (a transmission signal) transmitted from the capsule endoscope 2.

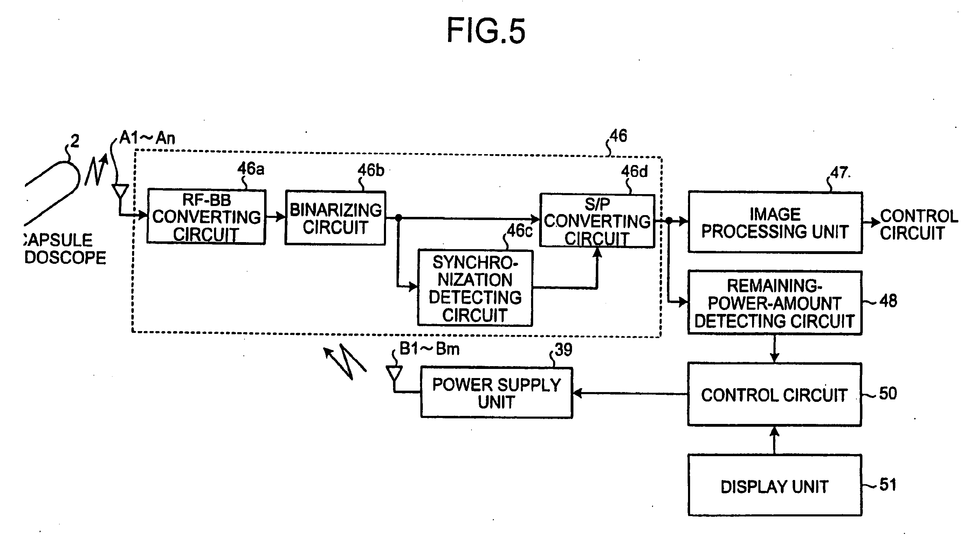

In the embodiment, as shown in FIG. 5, the RF receiving unit 46 includes: an RF-BB converting circuit 46a that converts an RF signal into a baseband signal; a binarizing circuit 46b that amplifies a baseband signal having a weak signal level and filters a frequency band, and generates a signal of a level that can be processed at a latter stage; a synchronization detecting circuit 46c that executes a synchronization detection based on an output signal of the binarizing circuit 46b; and a serial/parallel converting circuit 46d that converts an output signal of the binarizing circuit 46b from a serial signal into a parallel signal. The image information is stored into the portable recording medium 5 via the image processing unit 47 and the storage unit 49.

The external device 32 further includes: a remaining-power-amount detecting circuit 48 that detects information of a remaining power amount in the capacitor 22 of the capsule endoscope 2; a power supply unit 39 that supplies power based on the remaining-power-amount information from the control circuit 50; and a display unit 51 that displays an input remaining power amount. The remaining-power-amount detecting circuit 48 detects the remaining-power-amount information superimposed on the output signal of the serial/parallel converting circuit 46d, and outputs this information to the control circuit 50. The control circuit 50 changes the remaining-power-amount information detected by the remaining-power-amount detecting circuit 48 into a signal format that can be displayed on the display unit 51, and outputs the remaining-power-amount information to the power supply unit 39.

The display unit 51 has a configuration that directly displays information into a liquid crystal display such as an LCD, for example, and the display unit 51 directly displays a remaining power amount of the capacitor 22 of the capsule endoscope 2 input from the control circuit 50, on a screen of the LCD. The display unit 51 can be configured to output information to other medium such as a printer, as far as the user can recognize the information.

As shown in FIG. 4, the power supply unit 39 includes a superimposing circuit 43 connected to a latter stage of an oscillator 41. In the superimposing circuit 43, control information input from a control-information input unit 42 is superimposed on the power supply signal output from the oscillator 41. An amplifier circuit 44 connected to a latter stage of the superimposing circuit 43 amplifies the power supply signal on which the control information is superimposed, and outputs the amplified signal to a switching circuit 45. A power-supply control circuit 40 receives the remaining-power-amount information input from the control circuit 50, and changes an amplification rate of the power supply signal amplified by the amplifier circuit 44, based on the remaining power amount.

In other words, when the remaining-power-amount information from the control circuit 50 indicates a relatively large amount, for example, the power-supply control circuit 40 controls to suppress the amplification rate of the power supply signal. When the remaining-power-amount information indicates a relatively small amount, the power-supply control circuit 40 controls to increase the amplification rate of the power supply signal. The power supply signal input to the switching circuit 45 is radio transmitted from the power supply antenna of the transmitting/receiving jacket 31.

Assume that the control-information input unit 42 inputs an instruction to change the imaging rate of the CCD 13 from a first imaging rate to a second imaging rate. The superimposing circuit 43 superimposes a frame-number-change information signal transmitted from the control-information input unit 42, on the power supply signal, and inputs the superimposed signal to the amplifier circuit 44. The power-supply control circuit 40 controls to change the amplification rate, and the amplifier circuit 44 amplifies the signal at a predetermined amplification rate. The switching circuit 45 transmits the signal to an optimum power supply antenna among the power supply antennas B1 to Bm, and radio transmits the signal from the power supply antenna. The control-information input unit 42 according to the present embodiment can input various kinds of information. For example, the control-information input unit 42 can input: frame-number change information as information for changing an imaging rate of the CCD 13, that is, a number of frames imaged within a predetermined period of time; LED light-on time information for changing a light-on time and a light-on timing of the LED 11; and power supply ON/OFF information for switching the power supply of each unit between an active mode in which each unit acquires intra-subject information and a standby mode in which each unit stops acquiring the intra-subject information, by controlling the system control circuit 23.

As described above, the power receiving antenna 17 of the capsule endoscope 2 receives the radio-transmitted power supply signal. The frame-number change information signal as the control information signal separated from the power supply signal by the separating circuit 18 is input to a control-information detecting circuit 24. A frequency band of the control information signal is made different from a frequency band of the power supply signal. A frequency band of the control information signal is changed depending on the content of the control.

Accordingly, the receiving apparatus 3 can radio transmit a multiplexed signal. In the capsule endoscope 2, the separating circuit 18 can separate the input signal, based on the frequency band. The control-information detecting circuit 24 can detect the content of the control information. The control-information detecting circuit 24 which receives the control information signal detects that the signal is a frame-number change information signal, and controls the CCD driving circuit 14 to drive the CCD 13 at the second imaging rate, according to the control content (a change from the first imaging rate to the second imaging rate).

So long as the control-information input unit 42 does not input new information, the superimposing circuit 43 continues superimposing the same control information on the power supply signal, for at least a predetermined period of time. Therefore, even when there is a period when the capsule endoscope 2 cannot temporarily receive power, the capsule endoscope 2 can receive the control information. Because, after the insertion of the capsule endoscope 2 into the subject 1, the function of the capsule endoscope 2 can be radio controlled from the outside, it is possible to prevent acquiring too much intra-subject information of an unnecessary part, and to prevent careless consumption of power.

The control circuit 50 detects the received field strength during the image receiving period, by relating the strength to the selected one of the receiving antennas A1 to An. The control circuit 50 outputs an instruction signal to the power-supply control circuit 40 so as to switch to the power supply antenna disposed near the receiving antenna that receives the largest received field strength, among the power supply antennas B1 to Bm that transmit the power supply signal. The power-supply control circuit 40 switch controls the switching circuit 45, based on the instruction signal, and switches the power supply antenna that transmits the power supply signal.

As explained above, in the present embodiment, the remaining-power-amount information of the capacitor of the capsule endoscope is superimposed on a transmission signal, and the superimposed signal is transmitted to the receiving apparatus at the outside of the subject. The receiving apparatus detects the remaining-power-amount information, and displays the information on the display unit. Therefore, the user can promptly recognize the remaining power amount within the capsule endoscope.

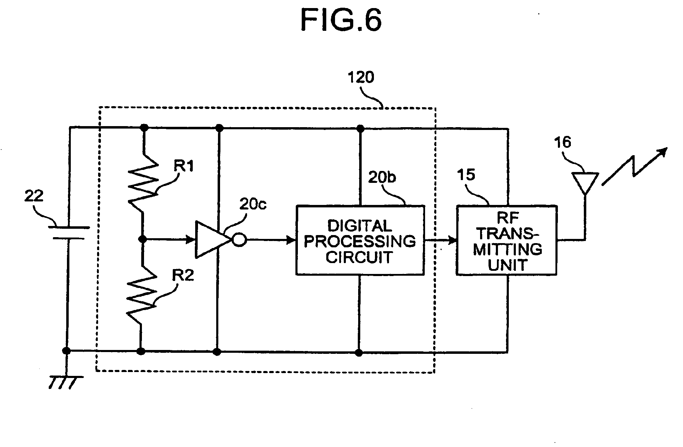

FIG. 6 is a block diagram of a configuration of a remaining-power-amount recognizing circuit 120 according to the invention, corresponding to the remaining-power-amount recognizing circuit 20 of the first embodiment shown in FIG. 2, and its periphery. The remaining-power-amount recognizing circuit 120 is different from the remaining-power-amount recognizing circuit 20 according to the first embodiment in that an inverter 20c is provided in place of the A/D converter, and that a predetermined target voltage of the remaining power amount of the capacitor 22 is resistance-divided to become close to a threshold value of the inverter 20c, and the resistance-divided voltage is input to the inverter 20c. When the divided voltage becomes lower than the threshold value of the inverter 20c, a high-level flag signal is output from the inverter 20c to the digital processing circuit 20b. The digital processing circuit 20b superimposes a signal that shows that the flag signal attains a high level, on the transmission signal, and outputs the superimposed signal to the RF transmitting unit 15.

On receiving a signal indicating that the flag signal attains a high level, the receiving apparatus 3 radio transmits a power supply signal at a constant amplification level to the capsule endoscope 2, thereby making it possible to externally charge the capsule endoscope 2.

As explained above, according to the present embodiment, a state of the remaining power amount in the capacitor of the capsule endoscope is determined according to a binary value of a high level and a low level, based on the threshold value of the inverter. Therefore, an A/D converter having a large circuit scale does not need to be used. Consequently, the circuit scale of the receiving apparatus can be made small, and weight can be decreased. As a result, the load of the subject can be decreased when the subject carries the receiving apparatus.

### INDUSTRIAL APPLICABILITY

As explained above, the intra-subject information acquiring system according to the present invention is useful for a medical observation apparatus that is inserted into the human body for an observation of an examined part of a subject, and is particularly suitable to promptly recognize a remaining power amount of a body-insertable apparatus.

## Claims

1. A body-insertable apparatus (2) comprising:
a function executing unit (11, 13, 23) adapted to execute a predetermined function of acquiring intra-subject information;
a power supply unit (20) adapted to supply power to the function executing unit (11, 13, 23);
a recognizing unit adapted to recognize a remaining power amount of the power supply unit (22),
said recognizing unit including
a voltage divider (R1, R2) adapted to divide a voltage of the power supply unit (22),
a remaining-power-amount notifying circuit (20c) connected to the voltage divider (R1, R2) and adapted to receive an input of the voltage divided by the voltage divider (R1, R2),
a superimposing unit (20b) which includes a digital processing circuit (20b) and is adapted to superimpose information of the remaining power amount recognized by the recognizing unit (20) on the transmission signal; wherein the digital processing circuit (20b) is connected to the remaining-power-amount notifying circuit (20c) and is adapted to receive an input from the remaining-power-amount notifying circuit (20c);
a transmitting unit (15, 16) adapted to transmit a transmission signal on which the information of the remaining power amount is superimposed by the superimposing unit (20b),
**characterized in that**
the transmission signal includes intra-subject information acquired by the function executing unit (11, 13, 23), and
the remaining-power-amount notifying circuit (20c) is adapted to output a signal of a predetermined level when the divided voltage becomes lower than a threshold value, and
the digital processing circuit (20b), on receiving an input of the signal of the predetermined level, is adapted to cause the transmitting unit (15, 16) to transmit a signal indicating that the remaining power is low.

2. The body-insertable apparatus (2) according to claim 1, further comprising:
a receiving antenna (17) adapted to receive a radio signal transmitted from a receiving apparatus (3);
a separating circuit (18) adapted to separate a power supply signal from the radio signal received by the receiving antenna (17);
a power reproducing circuit (19) adapted to reproduce the power supply signal as the power; and
a booster circuit (21) adapted to boost the power, wherein
the power supply unit (22) is adapted to store the boosted power.

3. The body-insertable apparatus (2) according to claim 1, wherein
the recognizing unit (20) includes
an analog-to-digital converter (20a) adapted to convert the divided voltage from an analog signal into a digital signal so that the remaining power amount is recognized.

4. The body-insertable apparatus (2) according to claim 3, wherein
the voltage divider (R1, R2) includes
a first resistor (R1) having a predetermined first resistance value and a second resistor (R2) having a predetermined second resistance value, one end of the first resistor (R1) being connected to one end of the power supply unit (22), the other end of the first resistor (R1) being connected to one end of the second resistor (R2) and the analog-to-digital converter (20a), the other end of the second resistor (R2) being connected to the other end of the power supply unit (22) and being grounded.

5. The body-insertable apparatus (2) according to claim 1, wherein
the remaining-power-amount notifying circuit (20c) includes an inverter (20c), and
the voltage divider (R1, R2) is adapted to divide a target voltage input from the power supply unit (22) so that the target voltage becomes close to a threshold value of the inverter (20c).

6. The body-insertable apparatus (2) according to claim 1, wherein
the voltage divider (R1, R2) includes a first resistor (R1) having a predetermined first resistance value and a second resistor (R2) having a predetermined second resistance value, one end of the first resistor (R1) being connected to one end of the power supply unit (18, 19, 21, 22), the other end of the first resistor (R1) being connected to one end of the second resistor (R2) and the remaining-power-amount notifying circuit (20c), the other end of the second resistor (R2) being connected to the other end of the power supply unit (22) and being grounded.

7. The body-insertable apparatus (2) according to claim 5, wherein
the voltage divider (R1, R2) includes a first resistor (R1) having a predetermined first resistance value and a second resistor (R2) having a predetermined second resistance value, one end of the first resistor (R1) being connected to one end of the power supply unit (18, 19, 21, 22), the other end of the first resistor (R1) being connected to one end of the second resistor (R2) and the inverter (20e), the other end of the second resistor (R2) being connected to the other end of the power supply unit (22) and being grounded.

8. The body-insertable apparatus (2) according to claim 1, wherein
the superimposing unit (20b) is adapted to superimpose only the information of the remaining power amount recognized by the recognizing unit (20) on the transmission signal.

9. The body-insertable apparatus (2) according to claim 1, wherein
the superimposing unit (20b) is adapted to superimpose the information of the remaining power amount recognized by the recognizing unit (20) on the transmission signal, together with information specific to the body-insertable apparatus (2).

10. The body-insertable apparatus (2) according to claim 9, wherein
the information specific to the body-insertable apparatus (2) is at least one of white-balance information and identification information of the body-insertable apparatus (2).

11. The body-insertable apparatus (2) according to claim 1, wherein
the superimposing unit (20b) is adapted to time-control a starting of the recognizing unit (20) so as to intermittently start the recognizing unit (20) to take in the information of the power remaining amount.

12. A receiving apparatus (3) comprising:
a receiving unit (A1-An, 46) adapted to receive a transmission signal including information of a remaining power amount of a body-insertable apparatus (2) and transmitted from the body-insertable apparatus (2), the body-insertable apparatus (2) being inserted into a subject body and acquiring intra-subject information;
a detector (48) adapted to detect the information of a remaining power amount of the body-insertable apparatus (2) from the transmission signal received by the receiving unit (A1-An, 46); and
a display unit (51) adapted to display the information of the remaining power amount detected by the detector (48), **characterized in that**
the transmission signal further includes intra subject-information, wherein
the receiving apparatus (3) further comprises a processing unit (47) that processes the intra-subject information included in the transmission signal received by the receiving unit (A1-An, 46), and wherein
the receiving unit (A1-An, 46) includes
a converting circuit (46a) adapted to convert the received RF transmission signal into a baseband signal,
a binarizing circuit (46a) adapted to process the baseband signal output from the converting circuit (46a),
a synchronization detecting circuit (46c) adapted to execute a synchronization detection based on an output signal of the binarizing circuit (46b), and
a serial/parallel converting circuit (46d) adapted to convert an output signal of the binarizing circuit (46b) from a serial signal into a parallel signal.

13. The receiving apparatus (3) according to claim 12 further comprising:
a power supply unit (39) adapted to generate a transmission signal based on the information of the remaining power amount detected by the detector (48); and
a transmitting antenna (B1-Bm) adapted to transmit the transmission signal generated by the power supply unit (44).

14. The receiving apparatus (3) according to claim 13 further comprising:
a controt-infonnation input unit (42) adapted to input predetermined control information for acquiring the intra-subject information, wherein
the power supply unit (39) includes
an oscillator (41) adapted to generate a power supply signal,
a superimposing circuit (43) adapted to superimpose the control information on the power supply signal output from the oscillator (41),
an amplifier (44) adapted to amplify the power supply signal on which the control information is superimposed, and
a power-supply controller (40) adapted to control to change an amplification rate of the power supply signal amplified by the amplifier based on the information of the remaining power amount, and wherein
the transmitting antenna (B1-Bm) is adapted to transmit the amplified power supply signal as the transmission signal.

15. The receiving apparatus (3) according to claim 12, wherein
the display unit (51) includes a liquid crystal display adapted to directly display the detected information of the remaining power amount.

16. The receiving apparatus (3) according to claim 12, wherein
the display unit (51) is adapted to further output the displayed information of the remaining power amount to a predetermined medium.

17. The receiving apparatus (3) according to claim 14, wherein
the power-supply controller (40) is adapted to control to suppress the amplification rate of the power supply signal when the information of the remaining power amount indicates a relatively large amount, and to control to increase the amplification rate of the power supply signal when the information of the remaining power amount indicates a relatively small amount.

## Patentansprüche

1. In einen Körper einführbare Vorrichtung (2), die umfasst:
eine Funktionsausführeinheit (11, 13, 23), die so eingerichtet ist, dass sie eine vorgegebene Funktion zum Erfassen intrakorporaler Information ausführt;
eine Energiezuführeinheit (20), die so eingerichtet ist, dass sie der Funktionsausführeinheit (11, 13, 23) Energie zuführt;
eine Erkennungseinheit, die so eingerichtet ist, dass sie eine verbleibende Energiemenge der Energiezuführeinheit (22) erkennt,
wobei die Erkennungseinheit enthält
einen Spannungsteiler (R1, R2), der so eingerichtet ist, dass er eine Spannung der Energiezuführeinheit (22) teilt,
eine Schaltung (20c) zum Informieren über eine verbleibende Energiemenge, die mit dem Spannungsteiler (R1, R2) verbunden und so eingerichtet ist, dass sie einen Eingang der durch den Spannungsteiler (R1, R2) geteilten Spannung empfängt,
eine Überlagerungseinheit (20b), die eine digitale Verarbeitungsschaltung (20b) enthält und so eingerichtet ist, dass sie dem Übertragungssignal eine Information über die durch die Erkennungseinheit (20) erkannte verbleibende Energiemenge überlagert; wobei die digitale Verarbeitungsschaltung (20b) mit der Schaltung (20c) zum Informieren über eine verbleibende Energiemenge verbunden und so eingerichtet ist, dass sie einen Eingang von der Schaltung (20c) zum Informieren über eine verbleibende Energiemenge empfängt;
eine Übertragungseinheit (15, 16), die so eingerichtet ist, dass sie ein Übertragungssignal überträgt, dem durch die Überlagerungseinheit (20b) die Information über die verbleibende Energiemenge überlagert wird,
**dadurch gekennzeichnet, dass**
das Übertragungssignal durch die Funktionsausführeinheit (11, 13, 23) erfasste intrakorporale Information enthält, und
die Schaltung (20c) zum Informieren über eine verbleibende Energiemenge so eingerichtet ist, dass sie ein Signal eines vorgegebenen Pegels ausgibt, wenn die geteilte Spannung unter einen Schwellenwert fällt, und
die digitale Verarbeitungsschaltung (20b) so eingerichtet ist, dass sie beim Empfangen des Signals des vorgegebenen Pegels die Übertragungseinheit (15, 16) veranlasst, ein Signal zu übertragen, das anzeigt, dass die verbleibende Energie gering ist.

2. In einen Körper einführbare Vorrichtung (2) nach Anspruch 1, die des Weiteren umfasst:
eine Empfangsantenne (17), die so eingerichtet ist, dass sie ein von einer Empfangsvorrichtung (3) übertragenes Funksignal empfängt ;
eine Trennschaltung (18), die so eingerichtet ist, dass sie ein Energiezuführsignal von dem durch die Empfangsantenne (17) empfangenen Funksignal trennt;
eine Energie-Reproduzierschaltung (19), die so eingerichtet ist, dass sie das Energiezuführsignal als die Energie reproduziert; und
eine Verstärkerschaltung (21), die so eingerichtet ist, dass sie die Energie verstärkt, wobei
die Energiezuführeinheit (22) so eingerichtet ist, dass sie die verstärkte Energie speichert.

3. In einen Körper einführbare Vorrichtung (2) nach Anspruch 1, wobei
die Erkennungseinheit (20) enthält:
einen Analog-Digital-Wandler (20a), der so eingerichtet ist, dass er die geteilte Spannung von einem analogen Signal in ein digitales Signal umwandelt, so dass die verbleibende Energiemenge erkannt wird.

4. In einen Körper einführbare Vorrichtung (2) nach Anspruch 3, wobei
der Spannungsteiler (R1, R2) enthält:
einen ersten Widerstand (R1), der einen vorgegebenen ersten Widerstandswert hat, sowie einen zweiten Widerstand (R2), der einen vorgegebenen zweiten Widerstandswert hat, wobei ein Ende des ersten Widerstandes (R1) mit einem Ende der Energiezuführeinheit (22) verbunden ist, das andere Ende des ersten Widerstandes (R1) mit einem Ende des zweiten Widerstandes (R2) und dem Analog-Digital-Wandler (20a) verbunden ist, und das andere Ende des zweiten Widerstandes (R2) mit dem anderen Ende der Energiezuführeinheit (22) verbunden und geerdet ist.

5. In einen Körper einführbare Vorrichtung (2) nach Anspruch 1, wobei
die Schaltung (20c) zum Informieren über eine verbleibende Energiemenge einen Inverter (20c) enthält, und
der Spannungsteiler (R1, R2) so eingerichtet ist, dass er eine Sollspannung, die von der Energiezuführeinheit (22) eingegeben wird, so teilt, dass die Sollspannung nahe an einem Schwellenwert des Inverters (20c) liegt.

6. In einen Körper einführbare Vorrichtung (2) nach Anspruch 1, wobei
der Spannungsteiler (R1, R2) einen ersten Widerstand (R1), der einen vorgegebenen ersten Widerstandswert hat, sowie einen zweiten Widerstand (R2) enthält, der einen vorgegebenen zweiten Widerstandswert hat, wobei ein Ende des ersten Widerstandes (R1) mit einem Ende der Energiezuführeinheit (18, 19, 21, 22) verbunden ist, das andere Ende des ersten Widerstandes (R1) mit einem Ende des zweiten Widerstandes (R2) und der Schaltung (20c) zum Informieren über eine verbleibende Energiemenge verbunden ist, und das andere Ende des zweiten Widerstandes (R2) mit dem anderen Ende der Energiezuführeinheit (22) verbunden und geerdet ist.

7. In einen Körper einführbare Vorrichtung (2) nach Anspruch 5, wobei
der Spannungsteiler (R1, R2) einen ersten Widerstand (R1), der einen vorgegebenen ersten Widerstandswert hat, sowie einen zweiten Widerstand (R2) enthält, der einen vorgegebenen zweiten Widerstandswert hat, wobei ein Ende des ersten Widerstandes (R1) mit einem Ende der Energiezuführeinheit (18, 19, 21, 22) verbunden ist, das andere Ende des ersten Widerstandes (R1) mit einem Ende des zweiten Widerstandes (R2) und dem Inverter (20e) verbunden ist, und das andere Ende des zweiten Widerstandes (R2) mit dem anderen Ende der Energiezuführeinheit (22) verbunden und geerdet ist.

8. In einen Körper einführbare Vorrichtung (2) nach Anspruch 1, wobei
die Überlagerungseinheit (20b) so eingerichtet ist, dass sie dem Übertragungssignal nur die Information über die durch die Erkennungseinheit (20) erkannte verbleibende Energiemenge überlagert.

9. In einen Körper einführbare Vorrichtung (2) nach Anspruch 1, wobei
die Überlagerungseinheit (20b) so eingerichtet ist, dass sie dem Übertragungssignal die Information über die durch die Erkennungseinheit (20) erkannte verbleibende Energiemenge zusammen mit für die in einen Körper einführbare Vorrichtung (2) spezifische Information überlagert.

10. In einen Körper einführbare Vorrichtung (2) nach Anspruch 9, wobei
die für die in einen Körper einführbare Vorrichtung spezifische Information eine Weißabgleich-Information oder/und eine Identifizierungs-Information der in einen Körper einführbaren Vorrichtung (2) ist/sind.

11. In einen Körper einführbare Vorrichtung (2) nach Anspruch 1, wobei
die Überlagerungseinheit (20b) so eingerichtet ist, dass sie eine Zeitsteuerung eines Startens der Erkennungseinheit (20) so durchführt, dass die Erkennungseinheit (20) intermittierend gestartet wird, um die Information über die verbleibende Energiemenge aufzunehmen.

12. Empfangsvorrichtung (3), die umfasst:
eine Empfangseinheit (A1-An, 46), die so eingerichtet ist, dass sie ein Übertragungssignal empfängt, das eine Information über eine verbleibende Energiemenge einer in einen Körper einführbaren Vorrichtung (2) enthält und von der in einen Körper einführbaren Vorrichtung (2) übertragen wird, wobei die in einen Körper einführbare Vorrichtung (2) in einen Körper eines Patienten eingeführt wird und intrakorporale Information erfasst;
einen Detektor (48), der so eingerichtet ist, dass er die Information über eine verbleibende Energiemenge der in einen Körper einführbaren Vorrichtung (2) aus dem durch die Empfangseinheit (A1-An, 46) empfangenen Übertragungssignal erfasst; und
eine Anzeigeeinheit (51), die so eingerichtet ist, dass sie die durch den Detektor (48) erfasste Information über die verbleibende Energiemenge anzeigt, **dadurch gekennzeichnet, dass**
das Übertragungssignal des Weiteren intrakorporale Information enthält, wobei
die Empfangsvorrichtung (3) des Weiteren eine Verarbeitungseinheit (47) umfasst, die die intrakorporale Information verarbeitet, die in dem durch die Empfangseinheit (A1-An, 46) empfangenen Übertragungssignal enthalten sind, und wobei
die Empfangseinheit (A1-An, 46) enthält:
eine Umwandlungsschaltung (46a), die so eingerichtet ist, dass sie das empfangene HF-Übertragungssignal in ein Basisbandsignal umwandelt,
eine Binärisierschaltung (46a), die so eingerichtet ist, dass sie das von der Umwandlungsschaltung (46a) ausgegebene Basisbandsignal verarbeitet,
eine Synchronisations-Erfassungsschaltung (46c), die so eingerichtet ist, dass sie eine Synchronisations-Erfassung auf Basis eines Ausgangssignals der Binärisierschaltung (46b) ausführt, und
eine Seriell-/Parallel-Umwandlungsschaltung (46d), die so eingerichtet ist, dass sie ein Ausgangssignal der Binärisierschaltung (46b) von einem seriellen Signal in ein paralleles Signal umwandelt.

13. Empfangsvorrichtung (3) nach Anspruch 12, die des Weiteren umfasst:
eine Energiezuführeinheit (39), die so eingerichtet ist, dass sie ein Übertragungssignal auf Basis der durch den Detektor (48) erfassten Information über die verbleibende Energiemenge erzeugt; und
eine Sendeantenne (B1-Bm), die so eingerichtet ist, dass sie das durch die Energiezuführeinheit (44) erzeugte Übertragungssignal sendet.

14. Empfangsvorrichtung (3) nach Anspruch 13, die des Weiteren umfasst:
eine Steuerinformations-Eingabeeinheit (42), die so eingerichtet ist, dass sie vorgegebene Steuerinformation zum Erfassen der intrakorporalen Information eingibt, wobei
die Energiezuführeinheit (39) enthält:
einen Oszillator (41), der so eingerichtet ist, dass er ein Energiezuführsignal erzeugt,
eine Überlagerungsschaltung (43), die so eingerichtet ist, dass sie dem von dem Oszillator (41) ausgegebenen Energiezuführsignal die Steuerinformation überlagert,
einen Verstärker (44), der so eingerichtet ist, dass er das Energiezuführsignal, dem die Steuerinformation überlagert ist, verstärkt und
eine Energiezuführ-Steuereinrichtung (40), die so eingerichtet ist, dass sie so steuert, dass ein Verstärkungsverhältnis des durch den Verstärker verstärkten Energiezuführsignals auf Basis der Information über die verbleibende Energiemenge geändert wird, und wobei
die Sendeantenne (B1-Bm) so eingerichtet ist, dass sie das verstärkte Energiezuführsignal als das Übertragungssignal sendet.

15. Empfangsvorrichtung (3) nach Anspruch 12, wobei
die Anzeigeeinheit (51) eine Flüssigkristall-Anzeigeeinrichtung enthält, die so eingerichtet ist, dass die die erfasste Information über die verbleibende Energiemenge direkt anzeigt.

16. Empfangsvorrichtung (3) nach Anspruch 12, wobei
die Anzeigeeinheit (51) so eingerichtet ist, dass sie des Weiteren die angezeigte Information über die verbleibende Energiemenge an ein vorgegebenes Medium ausgibt.

17. Empfangsvorrichtung (3) nach Anspruch 14, wobei
die Energiezuführ-Steuereinrichtung (40) so eingerichtet ist, dass sie so steuert, dass die Verstärkungsrate des Energiezuführsignals unterdrückt wird, wenn die Information über die verbleibende Energiemenge eine relativ große Menge anzeigt, und so steuert, dass die Verstärkungsrate des Energiezuführsignals erhöht wird, wenn die Information über die verbleibende Energiemenge eine relative kleine Menge anzeigt.

## Revendications

1. Appareil pouvant être inséré dans un corps (2) comprenant :
une unité d'exécution de fonction (11, 13, 23) adaptée pour exécuter une fonction prédéterminée consistant à acquérir des informations intra-sujet ;
une unité d'alimentation (20) adaptée pour alimenter l'unité d'exécution de fonction (11, 13, 23) ;
une unité de reconnaissance adaptée pour reconnaître une quantité de puissance restante dans l'unité d'alimentation (22),
ladite unité de reconnaissance comprenant
un diviseur de tension (R1, R2) adapté pour diviser une tension de l'unité d'alimentation (22),
un circuit de notification de quantité de puissance restante (20c) connecté au diviseur de tension (R1, R2) et adapté pour recevoir une entrée de la tension divisée par le diviseur de tension (R1, R2),
une unité de superposition (20b) qui comprend un circuit de traitement numérique (20b) et qui est adaptée pour superposer des informations de la quantité de puissance restante reconnue par l'unité de reconnaissance (20) sur le signal de transmission ; dans laquelle le circuit de traitement numérique (20b) est connecté au circuit de notification de quantité de puissance restante (20c) et est adapté pour recevoir une entrée provenant du circuit de notification de quantité de puissance restante (20c);
une unité de transmission (15, 16) adaptée pour transmettre un signal de transmission sur lequel les informations de la quantité de puissance restante sont superposées par l'unité de superposition (20b),
**caractérisé en ce que**
le signal de transmission comprend des informations intra-sujet acquises par l'unité d'exécution de fonction (11, 13, 23), et
le circuit de notification de quantité de puissance restante (20c) est adapté pour délivrer en sortie un signal d'un niveau prédéterminé lorsque la tension divisée devient inférieure à une tension de seuil, et
le circuit de traitement numérique (20b), sur réception d'une entrée du signal du niveau prédéterminé, est adapté pour amener l'unité de transmission (15, 16) à transmettre un signal indiquant que la puissance restante est basse.

2. Appareil pouvant être inséré dans un corps (2) selon la revendication 1, comprenant en outre :
une antenne de réception (17) adaptée pour recevoir un signal radio transmis depuis un appareil de réception (3) ;
un circuit de séparation (18) adapté pour séparer un signal d'alimentation du signal radio reçu par l'antenne de réception (17) ;
un circuit de reproduction de puissance (19) adapté pour reproduire le signal d'alimentation en tant que puissance ; et
un circuit d'amplification (21) adapté pour amplifier la puissance, dans lequel l'unité d'alimentation (22) est adaptée pour stocker la puissance amplifiée.

3. Appareil pouvant être inséré dans un corps (2) selon la revendication 1, dans lequel
l'unité de reconnaissance (20) comprend
un convertisseur analogique-numérique (20a) adapté pour convertir la tension divisée d'un signal analogique en un signal numérique de sorte que la quantité de puissance restante est reconnue.

4. Appareil pouvant être inséré dans un corps (2) selon la revendication 3, dans lequel
le diviseur de tension (R1, R2) comprend
une première résistance (R1) présentant une première valeur de résistance prédéterminée et une deuxième résistance (R2) présentant une deuxième valeur de résistance prédéterminée, une extrémité de la première résistance (R1) étant connectée à une extrémité de l'unité d'alimentation (22), l'autre extrémité de la première résistance (R1) étant connectée à une extrémité de la deuxième résistance (R2) et au convertisseur analogique-numérique (20a), l'autre extrémité de la deuxième résistance étant connectée à l'autre extrémité de l'unité d'alimentation (22) et étant mise à la masse.

5. Appareil pouvant être inséré dans un corps (2) selon la revendication 1, dans lequel
le circuit de notification de quantité de puissance restante (20c) comprend un inverseur (20c), et
le diviseur de tension (R1, R2) est adapté pour diviser une tension cible délivrée en entrée depuis l'unité d'alimentation (22) de sorte que la tension cible devient proche d'une valeur de seuil de l'inverseur (20c).

6. Appareil pouvant être inséré dans un corps (2) selon la revendication 1, dans lequel
le diviseur de tension (R1, R2) comprend une première résistance (R1) présentant une première valeur de résistance prédéterminée et une deuxième résistance (R2) présentant une deuxième valeur de résistance prédéterminée, une extrémité de la première résistance (R1) étant connectée à une extrémité de l'unité d'alimentation (18, 19, 21, 22), l'autre extrémité de la première résistance (R1) étant connectée à une extrémité de la deuxième résistance (R2) et au circuit de notification de quantité de puissance restante (20c), l'autre extrémité de la deuxième résistance (R2) étant connectée à l'autre extrémité de l'unité d'alimentation (22) et étant mise à la masse.

7. Appareil pouvant être inséré dans un corps (2) selon la revendication 5, dans lequel
le diviseur de tension (R1, R2) comprend une première résistance (R1) présentant une première valeur de résistance prédéterminée et une deuxième résistance (R2) présentant une deuxième valeur de résistance prédéterminée, une extrémité de la première résistance (R1) étant connectée à une extrémité de l'unité d'alimentation (18, 19, 21, 22), l'autre extrémité de la première résistance (R1) étant connectée à une extrémité de la deuxième résistance (R2) et à l'inverseur (20e), l'autre extrémité de la deuxième résistance (R2) étant connectée à l'autre extrémité de l'unité d'alimentation (22) et étant mise à la masse.

8. Appareil pouvant être inséré dans un corps (2) selon la revendication 1, dans lequel
l'unité de superposition (20b) est adaptée pour superposer seulement les informations de la quantité de puissance restante reconnue par l'unité de reconnaissance (20) sur le signal de transmission.

9. Appareil pouvant être inséré dans un corps (2) selon la revendication 1, dans lequel
l'unité de superposition (20b) est adaptée pour superposer les informations de la quantité de puissance restante reconnue par l'unité de reconnaissance (20) sur le signal de transmission, conjointement avec les informations spécifiques à l'appareil pouvant être inséré dans un corps (2).

10. Appareil pouvant être inséré dans un corps (2) selon la revendication 9, dans lequel
les informations spécifiques à l'appareil pouvant être inséré dans un corps (2) sont au moins l'une parmi des informations d'équilibre des blancs et des informations d'identification de l'appareil pouvant être inséré dans un corps (2).

11. Appareil pouvant être inséré dans un corps (2) selon la revendication 1, dans lequel
l'unité de superposition (20b) est adaptée pour commander dans le temps un démarrage de l'unité de reconnaissance (20) de façon à démarrer par intermittence l'unité de reconnaissance (20) pour recueillir les informations de la quantité restante de puissance.

12. Appareil de réception (3) comprenant :
une unité de réception (A1 à An, 46) adaptée pour recevoir un signal de transmission contenant des informations d'une quantité de puissance restante d'un appareil pouvant être inséré dans un corps (2) et transmis depuis l'appareil pouvant être inséré dans un corps (2), l'appareil pouvant être inséré dans un corps (2) étant inséré dans un corps de sujet et acquérant des informations intra-sujet ;
un détecteur (48) adapté pour détecter les informations d'une quantité de puissance restante de l'appareil pouvant être inséré dans un corps (2) à partir du signal de transmission reçu par l'unité de réception (A1 à An, 46) ; et
une unité d'affichage (51) adaptée pour afficher les informations de la quantité de puissance restante détectée par le détecteur (48), **caractérisé en ce que**
le signal de transmission comprend en outre des informations intra-sujet, dans lequel
l'appareil de réception (3) comprend en outre une unité de traitement (47) qui traite les informations intra-sujet contenues dans le signal de transmission reçu par l'unité de réception (A1 à An, 46), dans lequel
l'unité de réception (A1 à An, 46) comprend
un circuit de conversion (46a) adapté pour convertir le signal de transmission RF en un signal dans la bande de base,
un circuit de binarisation (46b) adapté pour traiter le signal dans la bande de base délivré en sortie du circuit de conversion (46a),
un circuit de détection de synchronisation (46c) adapté pour exécuter une détection de synchronisation sur la base d'un signal de sortie du circuit de binarisation (46b), et
un circuit de conversion série-parallèle (46d) adapté pour convertir un signal de sortie du circuit de binarisation (46b) d'un signal série en un signal parallèle.

13. Appareil de réception (3) selon la revendication 12, comprenant en outre :
une unité d'alimentation (39) adaptée pour générer un signal de transmission sur la base des informations de la quantité de puissance restante détectée par le détecteur (48) ; et
une antenne de transmission (B1-Bm) adaptée pour transmettre le signal de transmission généré par l'unité d'alimentation (44).

14. Appareil de réception (3) selon la revendication 13, comprenant en outre :
une unité d'entrée d'informations de commande (42) adaptée pour délivrer en entrée des informations de commande prédéterminées pour l'acquisition des informations intra-sujet, dans lequel
l'unité d'alimentation (39) comprend
un oscillateur (41) adapté pour générer un signal d'alimentation,
un circuit de superposition (43) adapté pour superposer les informations de commande sur le signal d'alimentation délivré en sortie depuis l'oscillateur (41),
un amplificateur (44) adapté pour amplifier le signal d'alimentation sur lequel les informations de commande sont superposées, et
un contrôleur d'alimentation (40) adapté pour commander le changement d'un taux d'amplification du signal d'alimentation amplifié par l'amplificateur sur la base des informations de la quantité de puissance restante, et dans lequel
l'antenne de transmission (B1-Bm) est adaptée pour transmettre le signal d'alimentation amplifié en tant que signal de transmission.

15. Appareil de réception (3) selon la revendication 12, dans lequel
l'unité d'affichage (51) comprend un affichage à cristaux liquides adapté pour afficher directement les informations détectées de la quantité de puissance restante.

16. Appareil de réception (3) selon la revendication 12, dans lequel
l'unité d'affichage (51) est adaptée pour en outre délivrer en sortie les informations affichées de la quantité de puissance restante sur un support prédéterminé.

17. Appareil de réception (3) selon la revendication 14, dans lequel
le contrôleur d'alimentation (40) est adapté pour commander la suppression du taux d'amplification du signal d'alimentation lorsque les informations de la quantité de puissance restante indiquent une quantité relativement importante, et pour commander l'augmentation du taux d'amplification du signal d'alimentation lorsque les informations de la quantité de puissance restante indiquent une quantité relativement faible.
